# EUROPEAN PATENT APPLICATION

(11) **EP 2 985 340 A1**
(43) Date of publication of application: **17.02.2016**
(21) Application number: 14782456.9
(22) Date of filing: 08.04.2014
(51) Int. Cl.: C12N 5/071, A61L 27/00, C12N 5/0789, C12N 5/10, C12N 15/09

(54) **METHOD FOR CULTURING HEPATOBLAST-LIKE CELLS AND CULTURE PRODUCT THEREOF**

(30) Priority: 08.04.2013 JP 2013080557
(71) Applicant: National Institutes of Biomedical Innovation, Health and Nutrition, Osaka 567-0085 (JP); Osaka University, Suita-shi, Osaka 565-0871 (JP)
(72) Inventor: MIZUGUCHI, Hiroyuki, Ibaraki-shi Osaka 567-0085 (JP); KAWABATA, Kenji, Ibaraki-shi Osaka 567-0085 (JP); TAKAYAMA, Kazuo, Ibaraki-shi Osaka 567-0085 (JP); SEKIGUCHI, Kiyotoshi, Suita-shi Osaka 565-0871 (JP)
(74) Representative: Paemen, Liesbet R.J.
(86) International application number: PCT/JP2014/060228
(87) International publication number: WO 2014/168157

(57) **Abstract**

Provided is a method of stably maintaining and culturing "hepatoblast-like cells" generated during a differentiation-inducing process from pluripotent stem cells to hepatocytes. The present invention also provides a culture product obtained by the culture method. The hepatoblast-like cells can be maintained and cultured stably by bringing a laminin into contact with the hepatoblast-like cells. The method of the present invention which uses a laminin makes it possible for the first time to culture, maintain, and proliferate the hepatoblast-like cells. Desired mature cells such as mature hepatocytes and bile duct epithelial cells can be generated in a short time period and can be acquired at a desired timing by maintaining the hepatoblast-like cells. Further, the resulting cultured hepatoblast-like cells were demonstrated to be capable of being induced to differentiate into mature hepatocytes, mature cholangiocytes, bile duct epithelial cells, and the like. Thus, a substantially pure culture product of the present invention can be utilized as a composition for transplantation for use in regeneration of hepatocytes and/or bile duct epithelial cells.

## Description

### Technical Field

The present invention relates to a method of culturing hepatoblast-like cells generated during a differentiation-inducing process from pluripotent stem cells (PSCs), such as induced pluripotent stem cells (iPS cells) or embryonic stem cells (ES cells), to hepatocytes, and to a culture product of the hepatoblast-like cells.

The present application claims priority from Japanese Patent Application No. 2013-080557, which is incorporated herein by reference.

### Background Art

Pluripotent stem cells are undifferentiated cells having pluripotency and a self-replication ability, and are suggested to have a tissue restoration ability after tissue damage. Thus, the pluripotent stem cells have been intensively investigated because the cells are useful in the fields of screening substances for treating various diseases and of regenerative medical treatments. Among the pluripotent stem cells, iPS cells are induced pluripotent stem cells which are generated by dedifferentiating somatic cells. Specifically, the iPS cells are generated by introducing genes encoding specific transcription factors, for example, OCT3/4, SOX2, KLF4, C-MYC, and the like into somatic cells, such as fibroblasts. In theory, cells having pluripotency can be induced to differentiate to all tissues and organs including liver and the like.

As methods of inducing differentiation from pluripotent stem cells to hepatocytes, a method involving forming cell aggregates (embryoid bodies), a method involving adding a humoral factor to a medium, and a method involving selectively employing an appropriate extracellular matrix, feeder cells, Matrigel, and the like have been mainly attempted. However, it has been reported that hepatocytes obtained by those methods have low activities of drug-metabolizing enzymes (Non Patent Literature 1 to 3). In order for stem cells to differentiate into mature hepatocytes, it is required that the stem cells undergo the steps of differentiation to mesendoderm, definitive endoderm cells, and hepatoblasts. In each step of the differentiation, humoral factors and compounds such as activin A, basic Fibroblast Growth Factor (bFGF), bone morphogenetic protein 4 (BMP4), fibroblast growth factor-4 (FGF-4), retinoic acid, or dimethyl sulfoxide (DMSO) have been used in culture systems. In addition, it has been reported that liver development requires transcription factors such as HEX, HNF4α, HNF6, and FOXA2 (Non Patent Literature 4).

There is a disclosure of a gene transfer method in the case of effective differentiation induction from stem cells, such as ES cells or iPS cells, to hepatocytes using a vector system for next-generation gene therapy (Patent Literature 4). In Patent Literature 4, there is a disclosure that stem cells, such as ES cells or iPS cells, can be effectively induced to differentiate into hepatocytes by introducing, for example, any one or more genes selected from HEX gene, HNF4α gene, HNF6 gene, and SOX17 gene into the stem cells by using an adenovirus vector. As described above, various investigations have been conducted on methods of inducing differentiation from pluripotent stem cells, such as ES cells or iPS cells, to hepatocytes effectively.

With regard to the culture of human pluripotent stem cells, there is a disclosure of a substrate for cell culture coated with E8 fragment of human laminin α5β1γ1 (human laminin 511) or E8 fragment of human laminin α3β2γ2 (human laminin 322) (Patent Literature 5). In Patent Literature 5, there is a disclosure that pluripotent stem cells are cultured in a feeder-free culture environment while their pluripotency is retained. However, in Patent Literature 5, there is a disclosure of the culture of human pluripotent stem cells, but there is no disclosure of tissue stem cells such as hepatoblast-like cells or cultured cells of intermediate cells of differentiation induction, and the application of the substrate for cell culture has been limited only to undifferentiated human pluripotent stem cells.

### Citation List

### Patent Literature

[PTL 1] JP 2002-272480 A (JP 3635462 B2)
[PTL 2] JP 2003-250566 A
[PTL 3] JP 2008-136381 A
[PTL 4] WO 2011/052504 A1
[PTL 5] JP 2011-78370 A

### Non Patent Literature

[NPL 1] Hepatology. 51, 297-305 [2010]
[NPL 2] PLoS One. 6, e24228 [2011]
[NPL 3] Hepatology. 55, 1193-203 [2012]
[NPL 4] Nature Reviews Genetics. 3, 499-512 [2002]

### Summary of Invention

### Technical Problem

An object of the present invention is to provide a method of stably maintaining and culturing"hepatoblast-like cells" generated during a differentiation-inducing process from pluripotent stem cells to hepatocytes. Another object of the present invention is to provide a culture product obtained by the culture method.

### Solution to Problem

The inventors of the present invention have conducted intensive investigations in order to achieve the above-mentioned objects, and as a result, found that the above-mentioned objects can be solved by contacting the hepatoblast-like cells generated during the differentiation-inducing process from pluripotent stem cells to hepatocytes with a laminin, and thus completed the present invention.

That is, the present invention includes the following.
1. A method of culturing hepatoblast-like cells, the method including the following steps of: bringing a laminin into contact with hepatoblast-like cells generated during a differentiation-inducing process from pluripotent stem cells to hepatocytes; and removing cells not attached to the laminin after bringing the laminin into contact with the hepatoblast-like cells.
2. A method of culturing hepatoblast-like cells according to the above-mentioned item 1, in which the laminin to be brought into contact with the hepatoblast-like cells generated during the differentiation-inducing process from pluripotent stem cells to hepatocytes is a laminin excluding laminin subtypes of laminin 2β1γ1 (laminin 211), laminin α4β1γ1 (laminin 411), and laminin α5β1γ1 (laminin 511) among laminin isoforms.
3. A method of culturing hepatoblast-like cells according to the above-mentioned item 1, in which the laminin to be brought into contact with the hepatoblast-like cells generated during the differentiation-inducing process from pluripotent stem cells to hepatocytes includes a laminin of an isoform having a property of exhibiting an affinity for integrin α6β1 higher than an affinity for integrin α3β1.
4. A method of culturing according to any one of the above-mentioned items 1 to 3, in which the laminin to be brought into contact with the hepatoblast-like cells generated during the differentiation-inducing process from pluripotent stem cells to hepatocytes includes a part or whole of laminin α1β1γ1 (laminin 111).
5. A method of culturing hepatoblast-like cells according to the above-mentioned item 4, in which the part of laminin 111 includes at least E8 fragment of laminin 111.
6. A method of culturing hepatoblast-like cells according to any one of the above-mentioned items 1 to 5, in which the hepatoblast-like cells include hepatoblast-like cells derived from induced pluripotent stem cells and/or collected embryonic stem cells.
7. A method of culturing hepatoblast-like cells according to the above-mentioned item 6, the method including the following steps of: 1) seeding the hepatoblast-like cells derived from induced pluripotent stem cells and/or collected embryonic stem cells on a solid support for cell culture coated with the laminin; 2) removing, in the solid support for cell culture on which the hepatoblast-like cells are seeded, non-adherent hepatoblast-like cells from the solid support; and 3) culturing the hepatoblast-like cells attached to the solid support in the step 2) on the laminin.
8. A method of culturing hepatoblast-like cells according to the above-mentioned item 7, the method further including the following steps of: 4) separating the hepatoblast-like cells obtained by culturing in the steps 1) to 3) of the above-mentioned item 7 from the solid support for cell culture, such that the hepatoblast-like cells are suspended in a medium; 5) seeding the suspended hepatoblast-like cells on a solid support for cell culture coated with the laminin, such that the number of cells is from 2.5×10² cells/cm² to 1.25×10⁵ cells/cm², preferably from 2.5×10³ cells/cm² to 2.5×10⁴ cells/cm², more preferably from 2×10⁴ cells/cm² to 2.5×10⁴ cells/cm²; and 6) culturing the hepatoblast-like cells attached to the solid support in the step 5) on the laminin.
9. A method of culturing hepatoblast-like cells according to the above-mentioned item 8, in which the steps 4) to 6) of the above-mentioned item 8 are repeated at least once.
10. A method of culturing hepatoblast-like cells according to any one of the above-mentioned items 7 to 9, the method including: dispersing the hepatoblast-like cells derived from induced pluripotent stem cells and/or collected embryonic stem cells to single cells, before the step 1) of the above-mentioned item 7 of seeding the hepatoblast-like cells on the solid support for cell culture coated with the laminin, and seeding the dispersed hepatoblast-like cells on the solid support for cell culture coated with the laminin of the step 1).
11. A substantially pure culture product of hepatoblast-like cells, including hepatoblast-like cells having an adhesion ability to laminin 111 than adhesion abilities to laminin 211, laminin 411, and laminin 511 among laminin isoforms.
12. A substantially pure culture product of hepatoblast-like cells, including hepatoblast-like cells expressing any one or more kinds of hepatoblast markers selected from ALB, CK19, CK7, and CYP3A7, and expressing integrin α6 and integrin β1.
13. A substantially pure culture product of hepatoblast-like cells, which is a culture product of hepatoblast-like cells expressing an integrin on surfaces thereof, the culture product including hepatoblast-like cells expressing integrin α6β1 more dominantly than integrin α3β1.
14. A substantially pure culture product, which is produced by the method of culturing hepatoblast-like cells of any one of the above-mentioned items 1 to 10.
15. A composition for transplantation for use in regeneration of hepatocytes and/or bile duct epithelial cells, the composition including the culture product of any one of the above-mentioned items 11 to 14 as an active ingredient.
16. A substrate for culturing hepatoblast-like cells, including a solid support for cell culture coated with a part or whole of laminin 111.
17. A substrate for culturing hepatoblast-like cells according to the above-mentioned item 16, in which the part of laminin 111 includes at least E8 fragment of laminin 111.
18. A pretreatment method for differentiation induction from hepatoblast-like cells to mature hepatocytes and/or bile duct epithelial cells, the method including the following steps of: bringing a laminin into contact with hepatoblast-like cells generated during a differentiation-inducing process from pluripotent stem cells to hepatocytes; and removing hepatoblast-like cells not attached to the laminin after bringing the laminin into contact with the hepatoblast-like cells.
19. A pretreatment method for differentiation induction from hepatoblast-like cells to mature hepatocytes and/or bile duct epithelial cells according to the above-mentioned item 18, in which the laminin to be brought into contact with the hepatoblast-like cells generated during the differentiation-inducing process from pluripotent stem cells to hepatocytes is a laminin excluding laminin subtypes of laminin 211, laminin 411, and laminin 511 among laminin isoforms.
20. A pretreatment method for differentiation induction from hepatoblast-like cells to mature hepatocytes and/or bile duct epithelial cells according to the above-mentioned item 18, in which the laminin to be brought into contact with the hepatoblast-like cells generated during the differentiation-inducing process from pluripotent stem cells to hepatocytes includes a laminin of an isoformhaving a property of exhibiting an affinity for integrin α6β1 higher than an affinity for integrin α3β1.
21. A pretreatment method for differentiation induction from hepatoblast-like cells to mature hepatocytes and/or bile duct epithelial cells according to the above-mentioned item 18, in which the laminin to be brought into contact with the hepatoblast-like cells generated during the differentiation-inducing process from pluripotent stem cells to hepatocytes includes a part or whole of laminin 111.
22. A pretreatment method for differentiation induction from hepatoblast-like cells to mature hepatocytes and/or bile duct epithelial cells according to the above-mentioned item 21, in which the part of laminin 111 includes at least E8 fragment of laminin 111.

### Advantageous Effects of Invention

The hepatoblast-like cells can be passaged by the culture method according to one embodiment of the present invention. It is difficult to passage the hepatoblast-like cells derived from ES cells and iPS cells. In the present invention, the number of passages is not particularly limited, but the hepatoblast-like cells can be passaged one or more times. The hepatoblast-like cells can maintain characteristics of the hepatoblast-like cells, and also can maintain differentiation potencies, even when passaged, for example, 3 or more times, 10 or more times, or 15 or more times repeatedly.

The cultured cells obtained as described above, in other words, the hepatoblast-like cells cultured by the culture method according to the embodiment of the present invention were positive for alpha-1-fetoprotein (AFP), Albumin (ALB), CK7, andCK19. Further, even when the hepatoblast-like cells were passaged many times, the expressions of integrin α6 and integrin β1 were observed. With regard to the hepatoblast-like cells which were passaged many times, the expressions of pan-hepatoblast markers CK8, CK18, EpCAM, and CD133, hepatoblast markers AFP, ALB, CYP3A7, and I-CAM, and the like were observed, and therefore, it was able to be confirmed that the hepatoblast-like cells were capable of being induced to differentiate to mature hepatocytes, bile duct epithelial cells, and the like. Further, by subjecting the cultured cells to further differentiation induction treatment, cells expressing CYP3A4, CYP2C9, CYP2C19, and α-1-antitrypsin (αAT), which are markers of mature hepatocytes, and cells expressing SOX9 and Type IV collagen, which are markers of bile duct epithelial cells, can be induced. In addition, a culture product obtained by the above-mentioned culture method, or mature hepatocytes or bile duct epithelial cells induced from the culture product, can be used as a composition for transplantation for use in the regeneration of hepatocytes and/or bile duct epithelial cells.

### Brief Description of Drawings

FIG. 1 is a diagram for illustrating a method of generating "hepatoblast-like cells" by treating an iPS cell line or an ES cell line (Reference Example 2).
FIG. 2 is a photograph for showing the morphology of cells obtained by the method illustrated in FIG. 1 on day 9 (Reference Example 2).
FIGS. 3 are photographs on the left-hand side for showing a group of cells in which the expression of AFP, which is a marker of hepatoblast-like cells (HBCs), is observed, and a graph on the right-hand side for showing that 80% of the cells are AFP positive (Reference Example 2).
FIG. 4 is a graph for showing results confirming, by real-time RT-PCR, the expression of each of genes of AFP, CD133, EpCAM, CK8, and CK18, which are markers of hepatoblast-like cells, in a group of cells on day 9 after starting culture (a group of cells containing both HBCs and NHBCs: before), a HBC group, and a NHBC group. In FIG. 4, a group of cells of which the expression of AFP was not observed in FIGS. 3 is shown as "non-hepatoblast-like cells: NHBCs" (Reference Example 2).
FIG. 5 is a schematic diagram for illustrating states of culturing hepatoblast-like cells on a laminin-coated solid support for cell culture. In FIG. 5, the cells which were attached to the solid support for cell culture are called as HBCs, and the cells which were not attached to the solid support for cell culture are called as NHBCs (Example 1).
FIG. 6 is a graph for showing results of measurement of the number of cells, after a group of cells on day 9 after starting culture (a group of cells containing both HBCs and NHBCs: before), HBCs, and NHBCs were seeded on various types of laminin-coated solid supports for cell culture (Example 1) .
FIG. 7 is a graph for showing results confirming the expressions of integrin α6 (ITGA6) and integrin β1 (ITGB1) by a group of cells on day 9 after starting culture (a group of cells containing HBCs and NHBCs: before), HBCs, and NHBCs (Example 1).
FIG. 8 is a graph for showing results of measurement of the culture period of time and the number of cultured cells when HBCs were cultured on various types of laminin-coated solid supports for cell culture (Example 1).
FIG. 9 is a graph for showing results confirming the expression of AFP in cells cultured on various types of laminin-coated solid supports for cell culture (Example 1).
FIG. 10 is a graph for showing results confirming the number of cells on a LN111-coated solid support for cell culture over time when cells were repeatedly subjected to passage operations for 15 times (Example 3).
FIGS. 11 are graphs for showing results confirming the expression of AFP, which is a marker of hepatoblast-like cells, in HBCs derived from ES cells (H9) and HBCs derived from iPS cells (Dotcom), when the cells were subjected to passage operations once (HBC P1), 10 times (HBC P10), and 15 times (HBC P15) on LN111-coated solid support for cell culture (Example 3).
FIGS. 12 are graphs for showing results of analyses of AFP, ALB, CK7, and CK19-expressing cells by a flow cytometry with regard to HBCs derived from ES cells (H9) without passage (HBC P0), the HBCs passaged once (HBC P1), and the HBCs passaged 10 times (HBC P10) (Example 4).
FIG. 13 is a graph for showing results confirming the expressions of integrin α6 (ITGA6) and integrin β1 (ITGB1) by a flow cytometry with regard to HBCs derived from ES cells (H9) without passage (HBC P0), the HBCs passaged once (HBC P1), and the HBCs passaged 10 times (HBC P10) (Example 4).
FIGS. 14 are graphs for showing results confirming the expression of each of marker genes of hepatic stem cells, pan-hepatoblasts, and hepatoblasts by real-time RT-PCR with regard to HBCs derived from ES cells (H9) without passage (HBC P0), the HBCs passaged once (HBC P1), and the HBCs passaged 10 times (HBC P10) (Example 4).
FIGS. 15 are a diagram for illustrating a method of inducing differentiation from HBCs derived from ES cells (H9) to hepatocytes, and graphs for showing results confirming the expression of each of marker genes by real-time RT-PCR, with regard to HBCs derived from ES cells (H9) without passage (HBC P0), the HBCs passaged 10 times (HBC P10), and HBCs derived from single cells (clone), after the cells were cultured for 14 days on Matrigel containing a medium which contains hepatocyte growth factor (HGF), oncostatin M (OsM), and dexamethasone (DEX) (Example 5).
FIGS. 16 and graphs for showing results confirming asialoglycoprotein receptor 1 (ASGR1) positive cell rates and amounts of secretion of ALB and Urea, with regard to the cells obtained by the method illustrated in FIGS. 15 (Example 5).
FIGS. 17 are a diagram for illustrating a method of inducing differentiation from HBCs derived from ES cells (H9) to bile duct epithelial cells, and a graph for showing results confirming the expression of each of marker genes by a real-time RT-PCR method, with regard to HBCs without passage (HBC P0), the HBCs passaged 10 times (HBC P10), and HBCs derived from single cells (clone), after the cells were cultured for 14 days by embedding the cells in collagen gel by using a medium containing epidermal growth factor (EGF) and an insulin-like growth factor (ILGF2) (Example 6).
FIG. 18 is a diagram for illustrating a mode of transplanting HBCs without passage (HBC P0) or the HBCs passaged 10 times (HBC P10) to immunodeficient mice with Liver injury (Example 7).
FIG. 19 is a graph for showing results of measurement of the secretion of human ALB into mouse serum two weeks after transplantation (Example 7).
FIGS. 20 are photographs for showing results confirming the expressions of ALB, AFP, and αAT two weeks after transplantation by an immunohistochemical staining method (Example 7).
FIG. 21 is a diagram for illustrating the differentiation induction from iPS cells or ES cells to HBCs and the maintenance and culture of HBCs (Example 8).
FIGS. 22 are a diagram for illustrating results of searching laminins suited for maintaining HBCs, and a graph for showing results confirming AFP positive rates by culturing cells on culture dishes coated with E8 fragments of various types of human laminins with different isoforms (Example 9).
FIGS. 23 are a diagram for illustrating results of searching laminins suited for maintaining HBCs, and a graph for showing results confirming cell proliferative capacities by culturing cells on culture dishes coated with E8 fragments of various types of human laminins with different isoforms (Example 9).
FIGS. 24 are a diagram and graph for showing results confirming properties of a culture product passaged 8 times by using a LN111- or LN111E8-coated culture dish by means of AFP positive rates (Example 10).
FIGS. 25 are a diagram and graph for showing results confirming properties of a culture product passaged by using a LN111- or LN111E8-coated culture dish by means of positive rates of markers except AFP (Example 10).
FIG. 26 is a graph for showing results confirming the expressions of various types of integrin' s genes by real-time RT-PCR with regard to HBCs and NHBCs on day 9 after starting culture (Example 11).

### Description of Embodiments

The present invention relates to a method of culturing hepatoblast-like cells, the method including: bringing a laminin into contact with hepatoblast-like cells generated during a differentiation-inducing process from pluripotent stem cells to hepatocytes, and also relates to a cultured product obtained by the culture method.

The "hepatoblast-like cells" as used herein refer to any intermediate cells to be generated during a differentiation-inducing process from endoderm derived from pluripotent stem cells to hepatocytes, and are not particularly limited. The hepatoblast-like cells as used herein include cells capable of differentiating into hepatocytes, for example, hepatic stem cells, juvenile hepatocytes, and the like, in addition to so-called hepatoblasts. The "pluripotent stem cells" as used herein may be undifferentiated cells having pluripotency and/or a self-replication ability, and are not particularly limited, but include pluripotent stem cells, for example, iPS cells, ES cells, and the like.

The "iPS cells" refer to induced pluripotent stem cells having pluripotency and proliferative capacity similar to ES cells. The "iPS cells" were generated by inducing reprogramming of differentiated cells without use of fertilized eggs, surplus embryos, or ES cells by introducing several kinds of genes into somatic cells, and were established from mouse fibroblasts in 2006 for the first time in the world. Further, it has been reported that human iPS cells were successfully established by introducing four human genes OCT3/4, SOX2, KLF4, and C-MYC homologous to the four genes used in establishing mouse iPS cells into fibroblasts derived from humans (Cell 131: 861-872, 2007). The iPS cells to be used in the present invention may be iPS cells generated by such a known method per se as the above-mentioned method or may be iPS cells to be generated by a novel method developed in the future.

The "ES cells" are pluripotent stem cells obtained by transferring a cell mass called an inner cell mass, which is generally present in the embryo at the blastocyst stage, to in vitro culture, followed by isolation as a population of undifferentiated stem cells from the culture. The ES cells were established as a cell line having pluripotency in mice by M.J.Evans & M.H.Kaufman (Nature, 292, 154, 1981), and then by G.R.Martin (Natl.Acad.Sci.USA, 78, 7634, 1981). There are many human-derived ES cell lines already established and these cell lines can be obtained from, for example, ES Cell International, Wisconsin Alumni Research Foundation, National Stem Cell Bank (NSCB), and the like. The ES cells are generally established by culturing early embryos. The ES cells may also be generated from early embryos containing the nuclei of somatic cells transplanted. Alternatively, there is a method involving generating a cell structure like the embryo at the blastocyst stage by transplanting the cell nuclei of a desired animal into cell vesicles (cytoplasts or ooplastoids) generated by dividing egg cells or denucleated egg cells of a foreign animal into a plurality of pieces and generating ES cells based on the cell structure. In addition, there have been reported an attempt to generate ES cells by developing parthenogenetic embryos to the phase similar to the blastocyst stage and generating ES cells from the embryos and a method involving generating ES cells having genetic information on the nuclei of somatic cells by fusion of ES cells and somatic cells. The ES cells to be used in the present invention may be ES cells generated by such a known method per se as the above-mentioned method or may be ES cells to be generated by a novel method developed in the future.

With regard to the differentiation-inducing process from pluripotent stem cells to hepatocytes in generating hepatoblast-like cells, a known method per se or any method developed in the future can be applied to the differentiation-inducing process by artificial treatment. As also described in the "Background Art" section, as a method of inducing differentiation from pluripotent stem cells to hepatocytes, a method involving forming cell aggregates (embryoid bodies), a method involving adding a humoral factor to a medium, and a method involving selectively employing an appropriate extracellular matrix, feeder cells, Matrigel, and the like may be applied (Non Patent Literatures 1 to 3). In order for stem cells to differentiate to mature hepatocytes, the stem cells generally need to differentiate via mesendoderm, definitive endoderm cells, and hepatoblasts, and in each differentiation step, a humoral factor or a compound, such as activin A, FGF-4, retinoic acid, or DMSO, can be used in the culture system. In addition, transcription factors such as HEX, HNF4α, HNF6, and FOXA2 have been reported to be required for the liver development (Non Patent Literature 5). Further, hepatocytes can be induced to be differentiated by introducing specific genes into stem cells, such as iPS cells or ES cells, by using a vector system. As the specific genes, there are given, for example, any one or more genes selected from HEX gene, HNF4α gene, HNF6 gene, and SOX17 gene (Patent Literature 4).

A laminin is one of the maj or proteins constituting the basement membrane and is considered to be involved in cell adhesion, migration, and proliferation in the basement membrane or the extracellular matrix. Among the major components constituting the basement membrane or the extracellular matrix, the laminin is said to be expressed from the earliest stage (2-cell stage). The structure of the laminin is a heterotrimer containing each one of α chain, β chain, and γ chain. There are 5, 4, and 3 kinds of α chains, β chains, and γ chains, respectively, and the laminin is designated by the combination of these chains. The laminin to be used in the present invention is a laminin or a derivative thereof of an isoform having a property of exhibiting an affinity for integrin α6β1 higher than an affinity for integrin α3β1. It is desired that the laminin have a property of exhibiting an affinity for integrin α6β1 at least 10 times or more as high as an affinity for integrin α3β1. The affinities of the laminin for integrin α3β1 and integrin α6β1 may be compared and evaluated by using a known test system which utilizes a cultured cell forced to express integrins, anti-integrin antibodies, and the like (Fujiwara, H et al. J. Biol Chem. 276, 17550-17558 [2001], Yao, CC et al. J. Biol.Chem. 271, 25598-25603 [1996] etc.).

Specifically, the laminin to be used in the present invention is a laminin excluding subtypes of laminin 211, laminin 411, and laminin 511 among laminin isoforms, and is preferably laminin 111. The origin of the laminin is not particularly limited, but the particularly suitable origin is a human. The laminin to be used in the present invention is not particularly limited, and may be a commercially available reagent of a full-length laminin, or may be one which is purified or prepared by a known method per se or a method developed in the future, or may be one which is generated by a technique such as gene recombination. The laminin to be used in the present invention is not necessarily a full-length laminin as long as the laminin has an active site of laminin. The active site of laminin refers to a site having a binding activity to an integrin, and is selected from C-terminal portions of laminin. Specifically, the active site of laminin refers to a site selected from C-terminal portions of laminins which are laminins excluding subtypes of laminin 211, laminin 411, and laminin 511 among laminin isoforms. More specifically, examples of the active site of laminin include sites of E8 fragments of laminins excluding subtypes of laminin 211, laminin 411, and laminin 511 among laminin isoforms, and a site in E8 fragment of laminin 111. A specific example of laminin 111 is laminin 111 formed of amino acid sequences specified by GenBank Accession No. (NP_005550 or NM_005559) (laminin α1), NP_002282 or NM_002291 (laminin β1), and NP_002284 or NM_002293 (laminin γ1), and an example of the active site of the laminin is E8 fragment of laminin 111 specified by α1E8 chain, β1E8 chain, and γ1E8 chain in the specified amino acid sequences based on its construction. E8 fragment may be specifically specified by Phe1878 to Gln2700 (α1E8 chain) in the sequence specified by GenBank Accession No. NM_005559 (laminin α1), Leu1561 to Leu1786 (β1E8 chain) in the sequence specified by NM_002291 (laminin β1), and Asn1364 to Pro1609 (γ1E8 chain) in the sequence specified by NM_002293 (laminin γ1) (Ido, H et al. J. Biol Chem. 282, 11144-111548 [2007], Ido, H et al. J. Biol.Chem. 283, 28149-28157 [2008]). It is suitable that the laminin which maybe used in the present invention include the site specified by α1E8 chain, β1E8 chain, and γ1E8 chain. In addition to the site specified by α1E8 chain, β1E8 chain, and γ1E8 chain, the laminin which may be used in the present invention may be a peptide or a protein formed of an amino acid sequence containing a part or whole of an amino acid sequence selected from amino acid sequences shown in GenBank Accession Nos. NM_005559 (laminin α1), NM_002291 (laminin β1), and NM_002293 (laminin γ1). On the other hand, the active site of the laminin may be shorter than E8 fragment as long as the site is selected from C-terminal portions and is capable of binding to an integrin. Accordingly, the active site of the laminin may be shorter than E8 fragment as long as the site has a binding activity to an integrin, in the laminin which may be used in the present invention, for example, laminins excluding subtypes of laminin 211, laminin 411, and laminin 511 among laminin isoforms, specifically laminin 111.

In this description, a method of culturing hepatoblast-like cells is more specifically a method including the following steps 1) to 3):
1) seeding the hepatoblast-like cells derived from iPS cells and/or ES cells on a solid support for cell culture coated with the laminin;
2) removing, in the solid support for cell culture on which the hepatoblast-like cells are seeded, non-adherent hepatoblast-like cells from the solid support; and
3) culturing the hepatoblast-like cells attached to the solid support in the step 2) on the laminin.

In this case, as a method of coating the solid support for cell culture with the laminin, a known method per se or any method developed in the future may be applied. For example, the solid support for cell culture may be coated with the laminin by diluting the laminin with a buffer solution, such as phosphate-buffered saline (PBS), to a concentration of from 10 µg/ml to 50 µg/ml, preferably from 15 µg/ml to 30 µg/ml, more preferably about 20 µg/ml to prepare Laminin Coating Solution (LCS), adding the Laminin Coating Solution onto the solid support for cell culture, and leaving the solid support for cell culture to stand still at from room temperature to about 37°C for from about 1 hour to about 12 hours. The coating concentration of the laminin on the solid support for cell culture is preferably from 0.5 µg/cm² to 500 µg/cm², more preferably from 1.5 µg/cm² to 100 µg/cm². Any support may be used as the solid support for cell culture of the present invention as long as the support can be used for cell culture, and examples thereof include a dish, multiwell dish, flask, plate, or microcarrier for culture made of glass or a plastic, and a polymer membrane such as a polyvinylidene fluoride membrane. In this description, the solid support for cell culture coated with the laminin of the present invention, or a dish, multiwell dish, flask, plate, or microcarrier for culture made of glass or a plastic, a polymer membrane such as a polyvinylidene fluoride membrane, a polymer membrane such as a polyvinylidene fluoride membrane, or the like, including the solid support for cell culture coated with laminin, is referred to as "substrate for culturing hepatoblast-like cells" and is encompassed in the scope of rights of the present invention. The "substrate for culturing hepatoblast-like cells" as used herein may be provided as a kit together with a necessary medium, reagent, and the like.

A method of culturing the hepatoblast-like cells on the solid support for cell culture coated with the laminin is described. The hepatoblast-like cells may be cultured by preparing a cell suspension by suspending the hepatoblast-like cells in a medium so that the number of cells is from 2.5×10² cells/cm² to 1.25×10⁵ cells/cm², and by seeding the cell suspension on the solid support for cell culture coated with the laminin. The culture may be performed in a feeder-free culture environment. The hepatoblast-like cells to be seeded may be blocked by an integrin α3 inhibitor beforehand, or the integrin α3 inhibitor may be added to the medium at the same time with starting the culture. The integrin α3 inhibitor is not particularly limited as long as the inhibitor blocks integrin α3 expressed in hepatoblast-like cells, and an example of the integrin α3 inhibitor is an anti-integrin α3 antibody.

The "medium" as used herein refers to a liquid or the like which contains nutritious ingredients necessary for the culture of cells, encompasses a so-called essential medium, and also encompasses a culture medium and the like which contain additives in addition to the essential medium. As a medium which may be used in the present invention, for example, media listed below may be used. An amount of a substance which is added to each medium may be appropriately increased or decreased depending on a purpose. The manufacturers/distributors of a reagent to be used are not limited to those described below as long as the reagent can exhibit a function equivalent to that described below.
(A) As a medium for maintaining an undifferentiated state of human ES/iPS cells, a medium for maintaining various types of stem cells including ReproStem (Reprocell Inc.), iPSellon (Cardio Inc.), TeSR-E8 (VERITAS Corporation), mTeSR (VERITAS Corporation), and the like supplemented with bFGF and the like may be used.
(B) As a medium for differentiation induction, a medium containing hESF-GRO (Cell Science & Technology Institute, Inc.), which is an essential medium for culturing ES cells, supplemented with insulin (10 µg/ml), transferrin (5 µg/ml), 2-mercaptoethanol (10 µM), 2-ethanolamine (10 µM), sodium selenite (20 nM), and bovine serum albumin (BSA) (1 mg/ml) may be used.
(C) As another mode of the medium for differentiation induction, a medium (FASEB J. 23: 114-22 (2009)) containing hESF-DIF (Cell Science & Technology Institute, Inc.), which is an essential medium for differentiation induction of ES cells, supplemented with insulin (10 µg/ml), transferrin (5 µg/ml), 2-mercaptoethanol (10 µM), 2-ethanolamine (10 µM), and sodium selenite (20 nM) may be used. In addition, a medium containing RPMI1640 medium (Sigma-Aldrich Co. LLC.) supplemented with 4 mM L-glutamine, B27 supplement (Invitrogen), and penicillin/streptomycin may also be used. Media to be used when endoderm and hepatoblast-like cells are subjected to differentiation induction are not limited to those described above as long as the media exhibit equivalent functions.
(D) As a medium for hepatoblast-like cells, DMEM/F12 medium may be used. The DMEM/F12 medium contains 10% FBS, insulin (10 µg/ml), transferrin (5 µg/ml), sodium selenite (20 nM), nicotinamide (10 mM), dexamethasone (DEX) (10⁻⁷M), HEPES (20 mM), NaHCO₃ (25 mM), L-glutamine (2 mM), penicillin/streptomycin, HGF (40 ng/ml), and EGF (20 ng/ml).

The hepatoblast-like cells obtained by culturing in the above-mentioned steps 1) to 3) may be cultured by passaging the cells in accordance with a method further including the following steps:
4) separating the hepatoblast-like cells obtained by culturing in the above-mentioned steps 1) to 3) from the solid support for cell culture, such that the hepatoblast-like cells are suspended in a medium;
5) seeding the suspended hepatoblast-like cells on a solid support for cell culture coated with the laminin, such that the number of cells is from 2.5×10² cells/cm² to 1.25×10⁵ cells/cm², preferably from 2.5×10³ cells/cm² to 2.5×10⁴ cells/cm², more preferably from 2×10⁴ cells/cm² to 2.5×10⁴ cells/cm² through the adjustment of the number of cells with a medium; and
6) culturing the hepatablast-like cells attached to the solid support in the step 5) on the laminin.

A culture product of the present invention can be obtained by culturing hepatoblast-like cells by the above-mentioned method, which enables the passage culture of hepatoblast-like cells. In the present invention, the number of times of passage culture is not particularly limited, but even when the above-mentioned steps 4) to 6) are repeated 1 or more times, for example, 3, 10, or 15 or more times, the hepatoblast-like cells can maintain the properties of the hepatoblast-like cells, and can maintain the ability to undergo the induction of differentiation. The present invention also extends to a culture product containing hepatoblast-like cells after passage culture, and to a substantially pure culture product. The hepatoblast-like cells after passage culture are hereinafter sometimes referred to as "cultured hepatoblast-like cells".

The "cultured hepatoblast-like cells" as used herein refer to hepatoblast-like cells obtained after undergoing passage operations 1 or more times, for example, 3, 10, or 15 or more times. Examples of the properties of cultured hepatoblast-like cells include expressions of any one or more kinds of hepatoblast markers selected from ALB, CK19, CK7, and CYP3A7, and expressions of integrin α6 and integrin β1. The "substantially pure culture product" refers to "cultured hepatoblast-like cells" specified as above, but the "substantially pure culture product" also means that it may include an ingredient of a medium and the like, in addition to the cultured hepatoblast-like cells. The "passage culture" refers to culturing cells by seeding the cells on a solid support for cell culture, culturing the cells in an appropriate medium for a certain period of time, and making the cells undergo operations such as exchange of the medium. The passage culture method per se is not limited to the culture method of the present invention, but, for example, refers to an operation involving bringing a laminin specified in this description into contact with "hepatoblast-like cells generated during a differentiation-inducing process from pluripotent stem cells to hepatocytes" specified in this description, removing cells not attached to the laminin, and thereafter, collecting cells attached to the laminin, and also refers to repeating such operation 1 or more times, for example, 3, 10, or 15 or more times.

The "substantially pure culture product" of the present invention maybe a culture product obtained by culturing hepatoblast-like cells by the culture method of the present invention, but is not limited to the culture product obtained by culturing hepatoblast-like cells by the culture method of the present invention, and may be a culture product having the following properties. The "substantially pure culture product" of the present invention may be a culture product containing passaged hepatoblast-like cells. For example, the "substantially pure culture product" of the present invention may be a culture product having a property of exhibiting an adhesion ability to laminin 111 higher than adhesion abilities to laminin 211, laminin 411, and laminin 511 among laminin isoforms. The "substantially pure culture product" of the present invention may be a culture product which expresses any one or more kinds of hepatoblast markers selected from ALB, CK19, CK7, and CYP3A7, and expresses integrin α6 and integrin β1, as long as the culture product contains passaged hepatoblast-like cells. The "substantially pure culture product" of the present invention may be a culture product which expresses integrins on their surfaces of cells, and expresses integrin α6β1 more dominantly than integrin α3β1, as long as the culture product contains passaged hepatoblast-like cells.

In this description, the expressions of integrin α6 and integrin β1 canbe confirmed by cultured hepatoblast-like cells after being passaged 1 or more times, even if the number of passages is increased further. In the cells which are passaged many times, for example, the expressions of pan-hepatoblast markers CK8, CK18, EpCAM, and CD133 and/or the expressions of hepatoblast markers AFP, ALB, CYP3A7, and I-CAM are observed. In addition, cells expressing CYP3A4, CYP2C9, CYP2C19, and αAT, which are markers of mature hepatocytes, and cells expressing SOX9 and Type IV collagen, which are markers of bile duct epithelial cells, can be induced, by further inducing the cultured hepatoblast-like cells to differentiate.

Accordingly,the hepatoblast-like cells,which are the culture product of the present invention, can differentiate into both hepatocytes and bile duct epithelial cells. Further, the differentiation to hepatocytes and the like can also be induced in vivo by transplanting the above-mentioned substantially pure culture product into an immunodeficient mouse with Liver injury. Thus, the culture product obtained by the above-mentioned culture method, or mature hepatocytes or bile duct epithelial cells induced from the culture product can be used as a composition for transplantation for use in the regeneration of hepatocytes and/or bile duct epithelial cells. In other words, the present invention also extends to a composition for transplantation for use in the regeneration of hepatocytes and/or bile duct epithelial cells containing the culture product of the present invention as an active ingredient.

In general, in the case of inducing pluripotent stem cells, such as human ES cells or iPS cells, to differentiate into hepatocytes or bile duct epithelial cells, a culture period of about 20 days is required. In addition, hitherto, it has been difficult to culture, maintain, and proliferate hepatoblast-like cells derived from iPS cells and/or ES cells, but the method of the present invention makes it possible for the first time to culture, maintain, and proliferate hepatoblast-like cells. Thus, desired mature cells, such as mature hepatocytes and bile duct epithelial cells, can be generated in a short time period and a predetermined number of cells can be acquired at a desired time by maintaining the hepatoblast-like cells. When the hepatoblast-like cells cultured by the culture method of the present invention are induced to differentiate into hepatocytes or bile duct epithelial cells, desired mature cells, such as mature hepatocytes and bile duct epithelial cells, can also be obtained by the culture for a time period of about from 10 days to 14 days. The in vivo toxicity of a pharmaceutical candidate compound can be predicted beforehand by adding the pharmaceutical candidate compound to the hepatocytes thus obtained, and analyzing the variations in expressions of markers of hepatotoxicity. Accordingly, a pharmaceutical candidate compound which should be excluded due to the problem of toxicity can be screened at an early stage, which leads to an expectation of the acceleration of drug discovery.

The present invention also extends to a pretreatment method for differentiation induction from hepatoblast-like cells to mature hepatocytes and/or bile duct epithelial cells. Specifically, the pretreatment method for differentiation induction from hepatoblast-like cells to mature hepatocytes and/or bile duct epithelial cells includes the steps of: bringing a laminin into contact with hepatoblast-like cells generated during a differentiation-inducing process from pluripotent stem cells to hepatocytes; and removing hepatoblast-like cells not attached to the laminin after bringing the laminin into contact with the hepatoblast-like cells. The laminin to be brought into contact with the hepatoblast-like cells generated during the differentiation-inducing process from pluripotent stem cells to hepatocytes maybe a laminin excluding laminin subtypes of laminin 211, laminin 411, and laminin 511 among laminin isoforms. In addition, the laminin may be a laminin of an isoform having a property of exhibiting an affinity for integrin α6β1 higher than an affinity for integrin α3β1.

The pretreatment for differentiation induction from hepatoblast-like cells to mature hepatocytes and/or bile duct epithelial cells is achieved by performing the step of bringing the laminin into contact with the hepatoblast like cells 1 or more times, and may be achieved by, for example, performing the step 3, 10, or 15 or more times.

### Examples

Now, the present invention is specifically described by way of Examples and Experimental Examples for further understanding of the present invention. However, it should be appreciated that the scope of the present invention is by no means limited to these Examples.

### (Reference Example 1) Compositions of Various Types of Media

Various types of media are required for human iPS cells or human ES cells in the culture method shown in Examples of the present invention. In Reference Example 1, compositions of media which can be used for various types of cultures are described.
(A) As a medium for maintaining an undifferentiated state of human ES/iPS cells, a medium for maintaining various types of stem cells including ReproStem (Reprocell Inc.), iPSellon (Cardio Inc.), TeSR-E8 (VERITAS Corporation), mTeSR (VERITAS Corporation) , and the like supplemented with bFGF and the like can be used. The medium is hereinafter referred to as "medium 1".
(B) As a medium for differentiation induction, a medium containing hESF-GRO (Cell Science & Technology Institute, Inc.), which is an essential medium for culturing ES cells, supplemented with insulin (10 µg/ml), transferrin (5 µg/ml), 2-mercaptoethanol (10 µM), 2-ethanolamine (10 µM), sodium selenite (20 nM), and bovine serum albumin (BSA) (1 mg/ml) may be used.
(C) As another mode of the medium for differentiation induction, a medium (FASEB J. 23: 114-22 (2009)) containing hESF-DIF (Cell Science & Technology Institute, Inc.), which is an essential medium for differentiation induction of ES cells, supplemented with insulin (10 µg/ml), transferrin (5 µg/ml), 2-mercaptoethanol (10 µM), 2-ethanolamine (10 µM), and sodium selenite (20 nm) may be used. The medium is hereinafter referred to as "medium 2". In addition, a medium containing RPMI1640 medium (Sigma-Aldrich Co. LLC.) supplemented with 4 mM L-glutamine, B27 supplement (Invitrogen), penicillin/streptomycin may also be used. Media to be used when endoderm and hepatoblast-like cells are subjected to differentiation induction are not limited to those described above as long as the media exhibit equivalent functions.
(D) As a medium for hepatoblast-like cells, DMEM/F12 medium may be used. The DMEM/F12 medium contains 10% FBS, insulin (10 µg/ml), transferrin (5 µg/ml), sodium selenite (20 nM), nicotinamide (10 mM), dexamethasone (DEX) (10⁻⁷M), HEPES (20 mM), NaHCO₃ (25 mM), L-glutamine (2 mM), penicillin/streptomycin, HGF (40 ng/ml), and EGF (20 ng/ml). The medium is hereinafter referred to as "medium 3".

### (Reference Example 2) Preparation of Hepatoblast-like Cells

In each of the following Examples, investigations were conducted by using hepatoblast-like cells (HBCs). In Reference Example 2, HBCs to be used in each of the following Examples are described. HBCs were generated by using an iPS cell line or ES cell lines, each of which is a cell line of pluripotent stemcells. The iPS cell line (Dotcom) was obtained from JCRB Cell Bank (JCRB Number: JCRB1327). The ES cell lines (H1 and H9) were handled according to the guidelines given by Ministry of Education, Culture, Sports, Science and Technology, and experiments were conducted after obtaining the approval by The Ethics Committee. The pluripotent stem cells were cultured together with mouse fetal fibroblasts (EMD Millipore Corporation) treated with mitomycin C by using the medium 1.

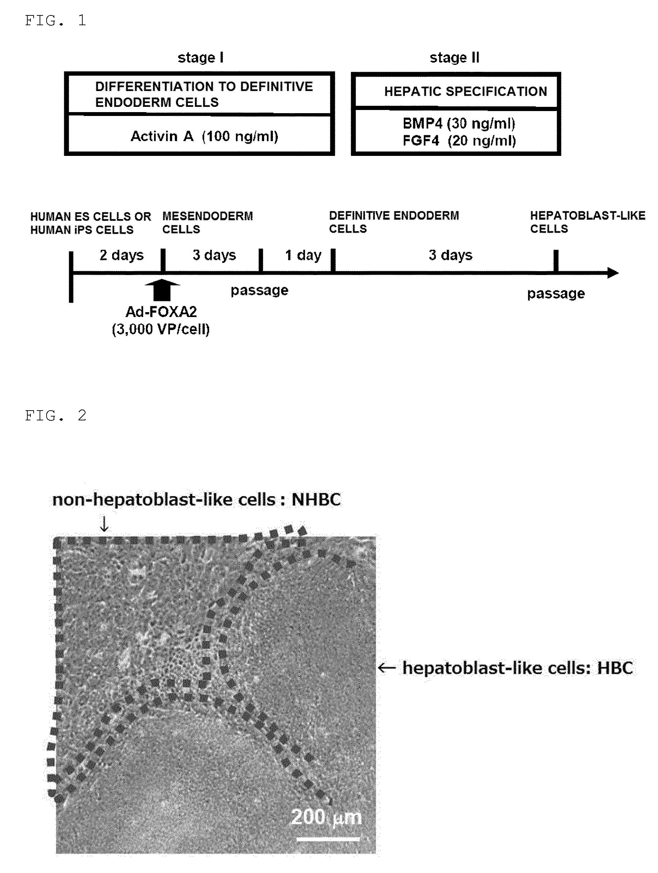

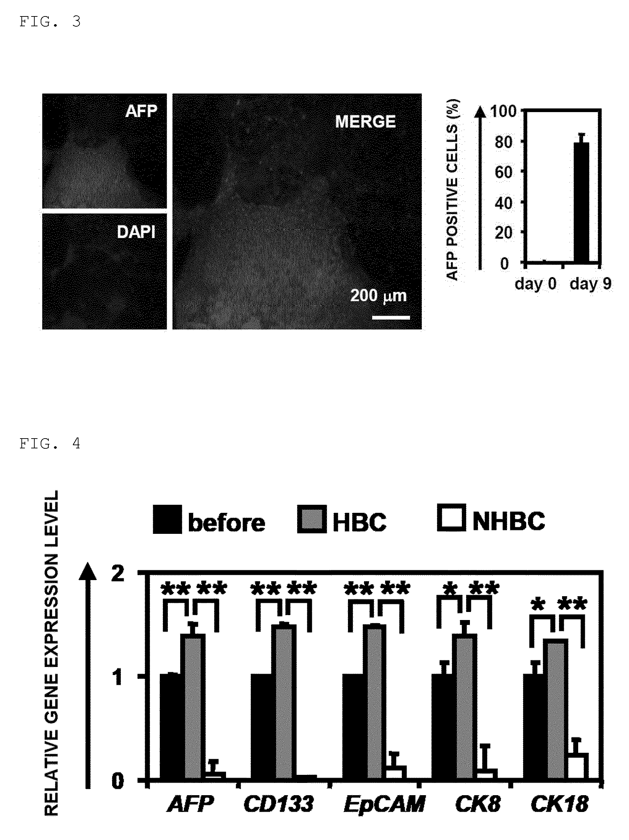

HBCs may be generated by conducting the differentiation induction treatment involving introducing differentiation induction factors, such as Activin A, and liver-related transcription factors, such as FOXA2 gene, according to the method disclosed in Patent Literature 4 (WO 2011/052504 A1) (FIG. 1) to an iPS cell line or an ES cell line (FIG. 2). In Reference Example 2, the ES cell line (H9) was used as a starting material and the medium 2 of Reference Example 1 was used as a medium for differentiation induction. By the differentiation induction treatment, a HBC group in which the expression of AFP, which was a marker of hepatoblast-like cells, was observed, was obtained as shown in the left-hand side of FIGS. 3. Eighty percent of the HBC cells obtained on day 9 shown in FIG. 1 was AFP-positive (see FIGS. 3). With regard to a group of cells before passage (a cell population which contained both HBCs and NHBCs: before), a HBC group, and a NHBC group, the expression of each of genes AFP, CD133, EpCAM, CK8, and CK18, which are markers of hepatoblast-like cells, was further confirmed. As a result, each of markers was expressed at a higher level in the isolated HBC group than in the group of cells before passage (the cell population which contained both HBCs and NHBCs: before) and in the NHBC group (FIG. 4).

### (Example 1, Comparative Example 1) Culture of Hepatoblast-like Cells

In Example 1 and Comparative Example 1, with regard to HBCs derived from ES cells obtained in Reference Example 2, properties of cells treated with laminins were confirmed. The coating treatment was performed on cell culture dishes by using various types of human laminins with different isoforms (laminin 111 (LN111), laminin 211 (LN211), laminin 411 (LN111), and laminin 511 (LN511)). The coating treatment was conducted by preparing 20 µg/ml of each Laminin Coating Solution, adding the Laminin Coating Solution to each well at 5 µg/cm², and leaving the cell culture dishes at room temperature for 1 hour or more.

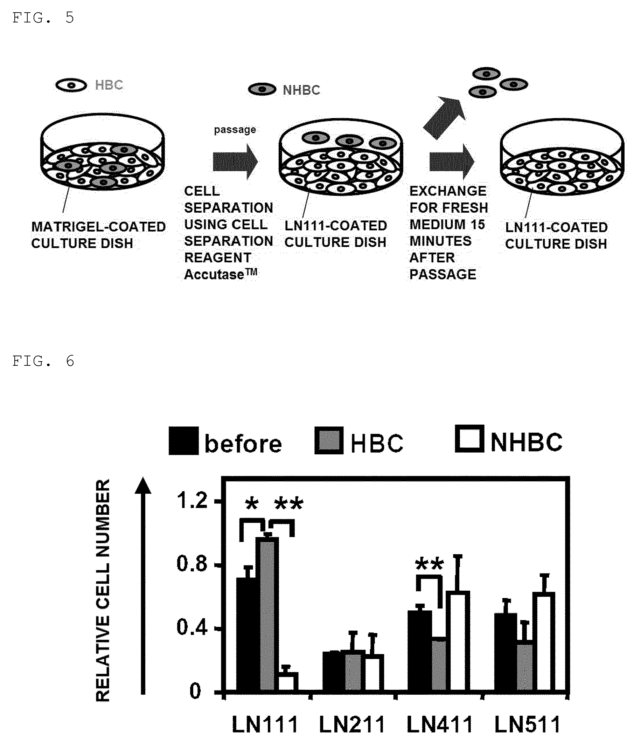

Cells before passage (the cell population which contained both HBCs and NHBCs: before), HBCs, and NHBCs were seeded on each well of the above-mentioned coated culture dish at 5×10³ cells/cm². The medium 3 of Reference Example 1 was used as a medium. As a result, it was confirmed that adhesion abilities of HBCs and NHBCs to various types of human laminins were different from each other (FIG. 6).

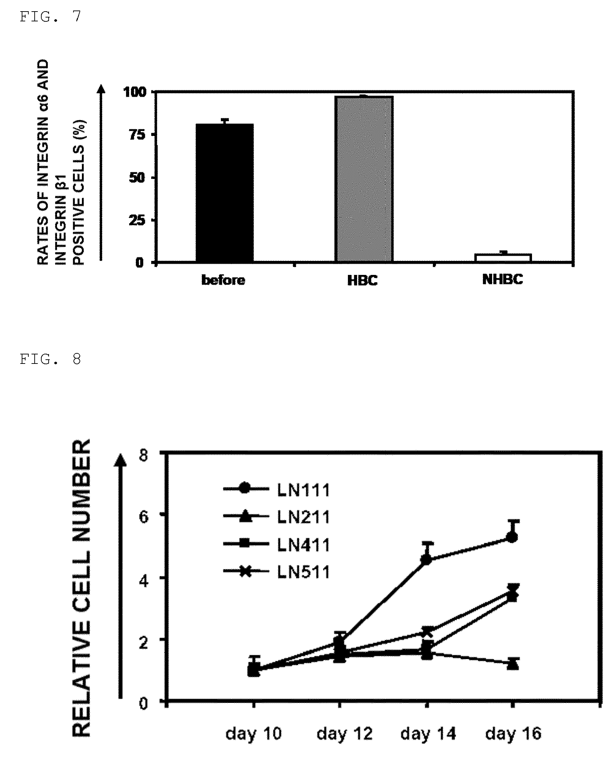

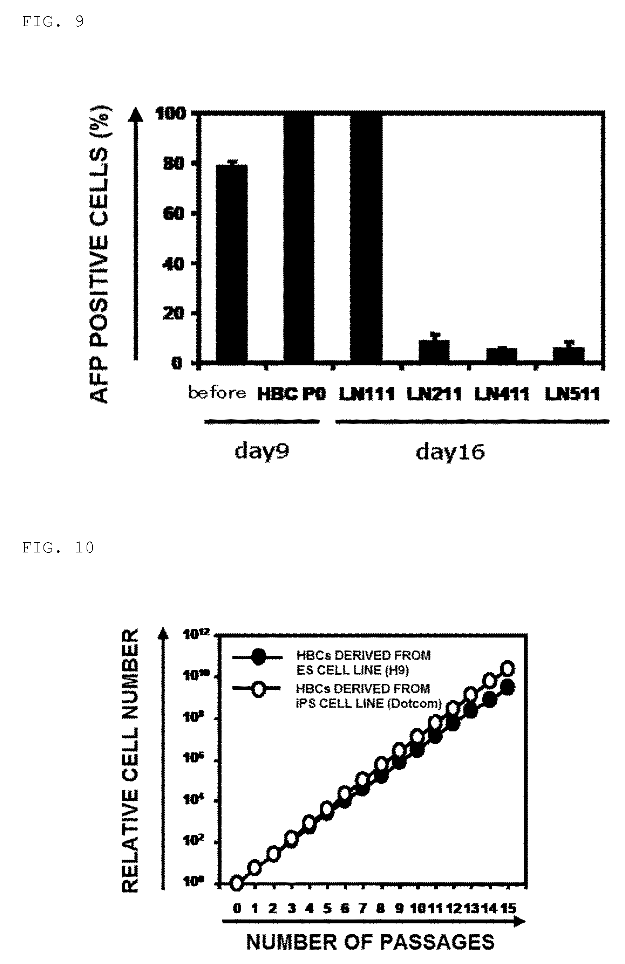

Next, the cells on day 9 after treated by the same procedure as in Reference Example 2 were seeded on each well of various types of laminin coated culture dishes at 7.5×10⁴ cells/cm², the medium was exchanged after 15 minutes, and cells attached to the dishes were used as HBCs and cells not attached to the dishes were used as NHBCs (see FIG. 5). With regard to cells before passage (the cell population which contained both HBCs and NHBCs : before), HBCs, and NHBCs, the expressions of integrin α6 (ITGA6) and integrin β1 (ITGB1) were confirmed. As a result, almost all cells of HBCs were confirmed to be co-positive (FIG. 7). The HBCs attached to the dishes were each further cultured for 7 days. As a result, it was confirmed that the proliferation rate was highest in cells cultured in a LN111-coated culture dish (FIG. 8). Further, with regard to cells cultured in various types of laminin-coated culture dishes, the expression of AFP was confirmed. As a result, it was confirmed that the cells cultured in the LN111-coated culture dish expressed AFP in a similar manner to a group of cells immediately after passage (P0) (see FIG. 9). In each of the following Examples and Comparative Examples, the medium 3 of Reference Example 1 was used for culturing HBCs and NHBCs.

### (Example 2 and Comparative Example 2) Properties of Hepatoblast-like Cells

In Example 2 and Comparative Example 2, HBCs derived from ES cells (H9) obtained in Reference Example 2 were seeded in each well of various types of laminin-coated culture dishes at 200 cells/cm² in a similar manner to Example 1 and Comparative Example 1, the HBCs were cultured by using the medium 3, and the expressions of ALB and CK7 by cultured cells were confirmed. The HBCs were fixed by using a 4% paraformaldehyde solution 5 days after seeding the HBCs, immunostaining was performed, and thereafter, the numbers of colonies which were ALB positive (+)/CK7 positive (+), ALB positive (+)/CK7 negative (-) and ALB negative (-) /CK7 negative (+) were counted. The results are shown in Table 1. When the cells were cultured in the LN111-coated culture dish, the highest number of the ALB positive (+)/CK7 positive (+) colonies, which were colonies of hepatoblast-like cells, was able to be confirmed.

**[Table 1]**

| Human recombinant laminin | ALB+/CK7+ | ALB+/CK7- | ALB-/CK7+ |
|---|---|---|---|
| LN111 | 82 | 2 | 5 |
| LN211 | 25 | 3 | 12 |
| LN411 | 12 | 6 | 31 |
| LN511 | 14 | 2 | 36 |

### (Example 3) Culture of Hepatoblast-like Cells

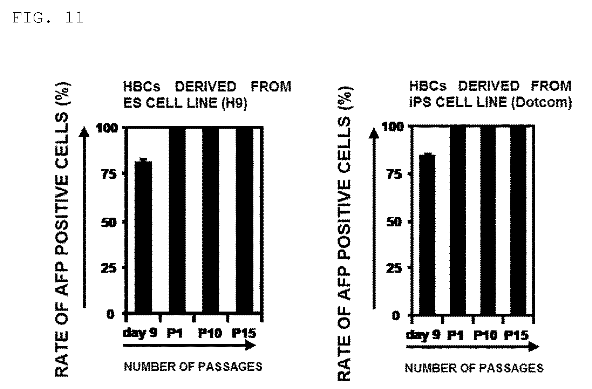

In Example 3, the proliferative capacities of HBCs derived from ES cells (H9) obtained in Reference Example 2 and HBCs derived from iPS cells (Dotcom) generated by the same procedure were confirmed. The cells derived from ES cells or those derived from iPS cells on day 9 obtained by treating the cells by the same procedure as in Reference Example 2 were seeded in each well of the LN111-coated culture dish at 7.5×10⁴ cells/cm², and the medium was exchanged after 15 minutes (the number of times of passage was 0, on day 9). After that, the cells were cultured for from 6 days to 7 days with the medium being exchanged every day, a cell suspension was prepared so that the number of cells was 7.5×10⁴ cells/cm², and the cells were passaged on the LN111-coated culture dish. Such passage operations were performed 15 times. As a result, the cultured cells continued to proliferate even after passaged 15 times (P15) (see FIG. 10). With regard to each of cells passaged once (HBC P1), 10 times (HBC P10), and 15 times (HBC P15), AFP positive cells were confirmed by flow cytometry analysis, and it was confirmed that all the cells exhibited high expression rates of AFP regardless of the number of passages (FIGS. 11).

### (Example 4) Abilities to undergo Induction of Differentiation (1) of Cultured Hepatoblast-like Cells

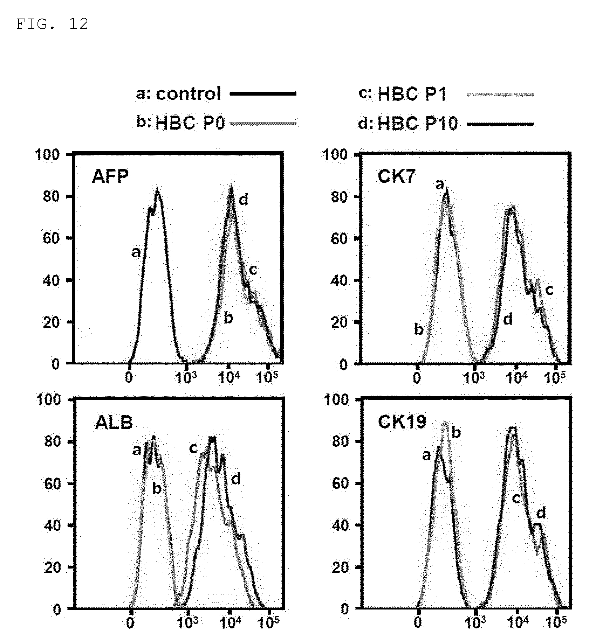

With regard to each of HBCs derived from ES cells (H9) without passage (HBC P0) the HBCs passaged once (HBC P1), and the HBCs passaged 10 times (HBC P10) in the same procedure as in Example 3, the expressions of AFP, ALB, CK7, and CK19 were analyzed by flow cytometry. Unstained cells were used as a control. As a result, the expressions of ALB, CK7, and CK19 in the cells with the number of passages of 0 were equivalent to the control, and their expressions were confirmed in the cells with the number of passages of 1 and the number of passages of 10. It was confirmed that AFP was similarly expressed in all the cells regardless of the number of passages (FIGS. 12).

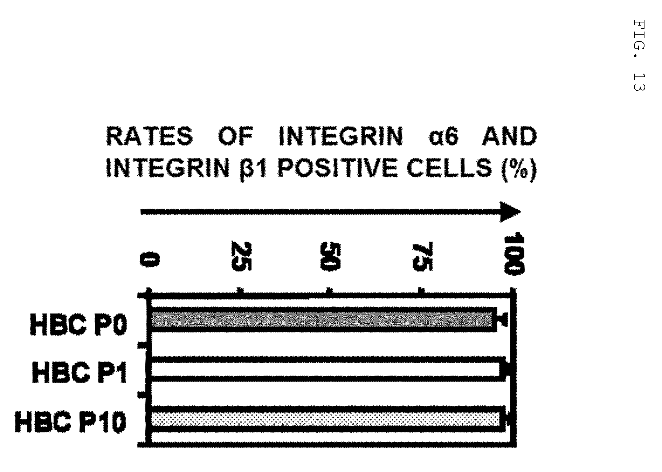

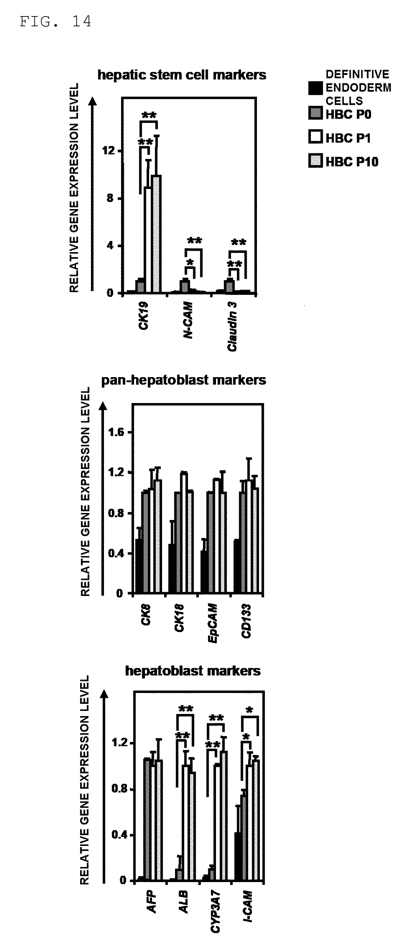

In addition, with regard to each of the cells, the expression of each of integrin α6 (ITGA6) and integrin β1 (ITGB1) was confirmed by flow cytometry. As a result, in all the cases where the numbers of passages were 0, 1, and 10, it was confirmed that positive rates of the expressions of ITGA6 and ITGB1 were high (FIG. 13). Unstained cells were used as a control, and the positive rate of each gene in the control cells was specified as 0%. Further, with regard to each of the cells, the gene expressions of hepatic stem cell markers CK19, N-CAM, and Claudin 3, pan-hepatoblast markers CK8, CK18, EpCAM, and CD133, and hepatoblast markers AFP, ALB, CYP3A7 and I-CAM were confirmed by a real-time RT-PCR technique (FIGS. 14). Various types of commercially available primer sets and reagents were used for real-time RT-PCR measurement, and the measurement was performed by using GAPDH as a housekeeping gene. From the results, it was shown that the HBCs derived from ES cells (H9) had a gene expression profile similar to that of hepatoblasts which was previously reported.

### (Example 5) Abilities to undergo Induction of Differentiation (2) of Cultured Hepatoblast-like Cells

From the results of Example 4, the HBCs derived from ES cells (H9) were suggested to be capable of differentiating to both hepatocytes and bile duct epithelial cells. In view of the foregoing, in Example 5, HBCs (H9) with the number of passages of 0 (HBC P0), HBCs (H9) with the number of passages of 10 (HBC P10), and HBCs derived from a single cell (HBC clone) were cultured in a medium containing HGF, OsM (20 ng/ml), and DEX (10⁻⁶ M) on Matrigel for 14 days, and the expression of each of marker genes before and after the differentiation induction treatment into liver by HGF, OsM, and DEX was confirmed. Clone cells were obtained by seeding cells on a culture dish at 200 cells/cm². The expression of each of gene markers was confirmed by real-time RT-PCR. Commercially available primer sets and reagents were used for real-time RT-PCR measurement. CYP3A4, CYP2C9, CYP2C19, and αAT are markers of mature hepatocytes, and CK7 and CK19 are markers of bile duct epithelial cells.

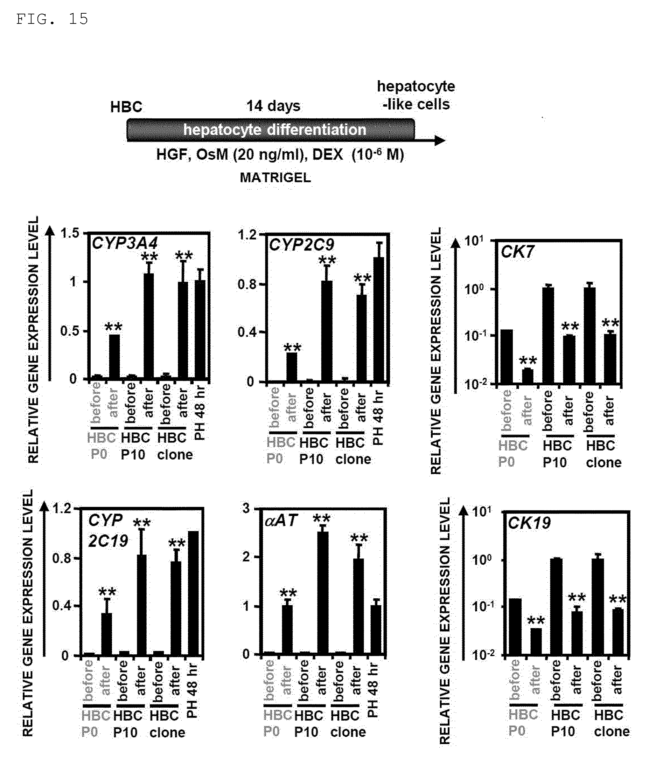

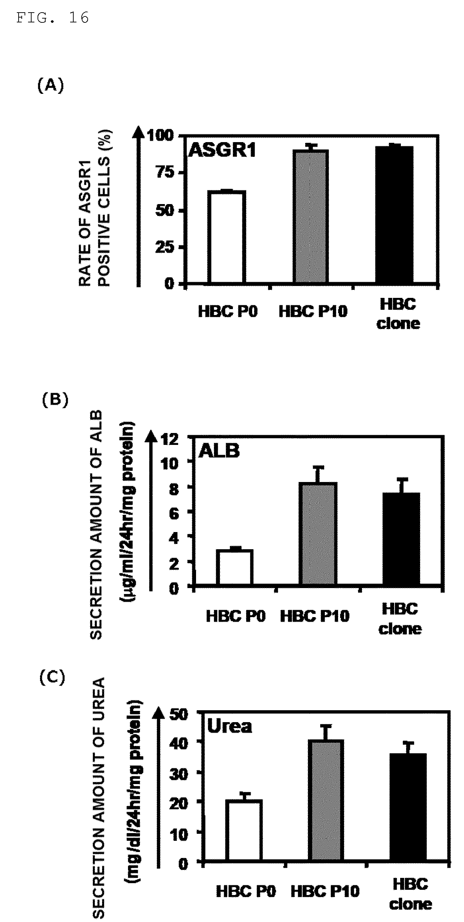

Each of relative expression levels of marker genes of mature hepatocytes was confirmed by defining the expression level by human frozen hepatocytes cultured for 48 hours after being seeded on a culture dish as 1. Similarly, each of relative expression levels of marker genes of bile duct epithelial cells was confirmed by defining the expression level in cells of HBC P10 before being subjected to the differentiation induction as 1. Based on the above, markers of mature hepatocytes were expressed at higher levels in all the cells after being subj ected to the differentiation induction to liver as compared to those before being subjected to the differentiation induction to liver. Further, it was confirmed that expression levels of marker genes of mature hepatocytes (CYP3A4, CYP2C9, CYP2C19, and αAT) were higher in the hepatocytes obtained by differentiation induction on the HBCs (H9) with the number of passages of 10 (HBC P10) than those in the hepatocytes obtained by differentiation induction on HBCs purified only with LN111 (HBC P0) (FIGS. 15). From these results, it was suggested that the HBCs (H9) were capable of differentiating into hepatocytes. It was confirmed that the differentiation rate into mature hepatocytes of hepatocytes obtained by differentiation induction on the HBCs (H9) with the number of passages of 10 (HBC P10) was higher than that of the hepatocytes obtained by differentiation induction on the HBCs (HBC P0), also from the ASGR1 positive cell rates analyzed by flow cytometry (FIG. 16A) and the amounts of ALB secretion and Urea secretion (FIGS. 16B and 16C).

According to the above results, it was suggested that the differentiation efficiency and differentiation ability to hepatocytes were improved by maintaining the HBCs on LN111.

### (Example 6) Abilities to undergo Induction of Differentiation (2) of Cultured Hepatoblast-like Cells

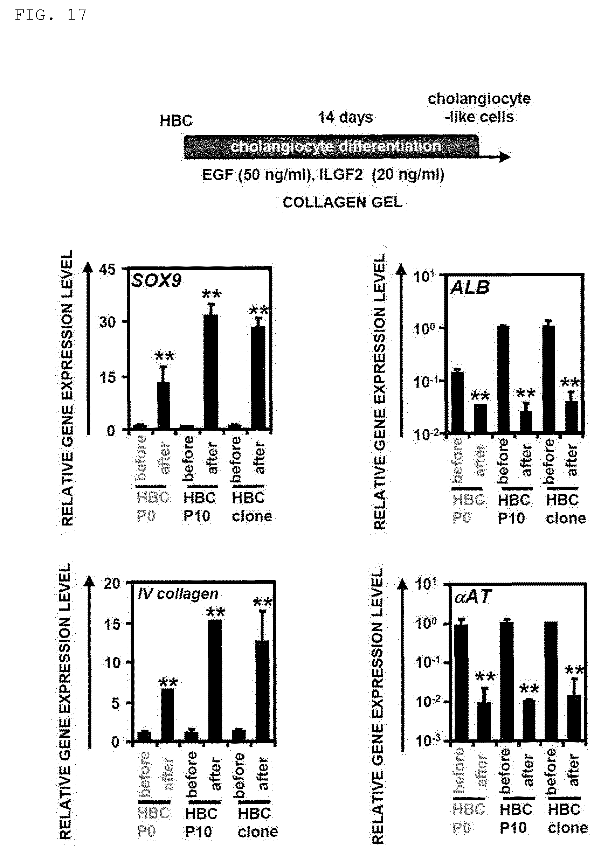

It was suggested from the results of Example 4 that HBCs derived from ES cells were capable of differentiating to both hepatocytes and bile duct epithelial cells, and hence the ability of HBCs to be induced to differentiate to bile duct epithelial cells was confirmed in Example 6. Specifically, in Example 6, HBCs (H9) without passage (HBC P0), HBCs passaged 10 times (HBC P10), and HBCs derived from a single cell (clone) were embedded in collagen gel by using a medium containing EGF (50 ng/ml) and ILGF2 (20 ng/ml), and were cultured for 14 days. Then, the expression of each of marker genes before and after the differentiation induction treatment to a bile duct by EGF and ILGF2 was confirmed. The expression was confirmed by a RT-PCR technique (FIGS. 17). Commercially available primer sets and reagents were used for RT-PCR measurement. SOX9 and Type IV collagen are markers of bile duct epithelial cells, and ALB and αAT are markers of mature hepatocytes.

Each of relative expression levels of marker genes of bile duct epithelial cells and mature hepatocytes was confirmed by defining the expression level in cells of HBC P10 before being subjected to the differentiation induction as 1. The expressions of markers of bile duct epithelial cells were increased by the differentiation induction treatment. In addition, the expression levels of bile duct marker genes (SOX9 and Type IV collagen) by bile duct epithelial cells which were induced to differentiate from HBC P10 were higher than those which were induced to differentiate from HBC P0 (FIGS. 17). From the above-mentioned results, it is suggested that HBCs derived fromES cells (H9) have the differentiation ability to bile duct epithelial cells.

According to the above-mentioned results, it was suggested that the differentiation ability to bile duct epithelial cells was improved by maintaining the HBCs on LN111.

### (Example 7) Abilities to undergo Induction of Differentiation (3) of Cultured Hepatoblast-like Cells (in vivo)

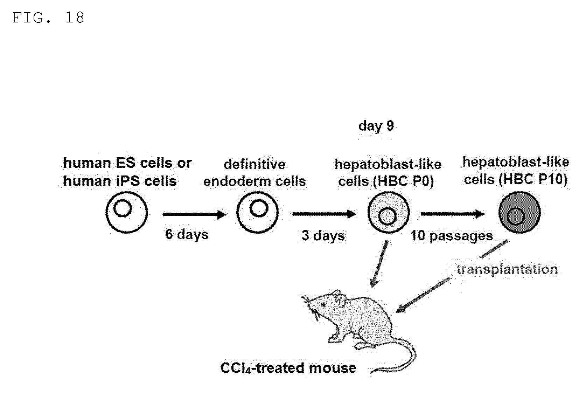

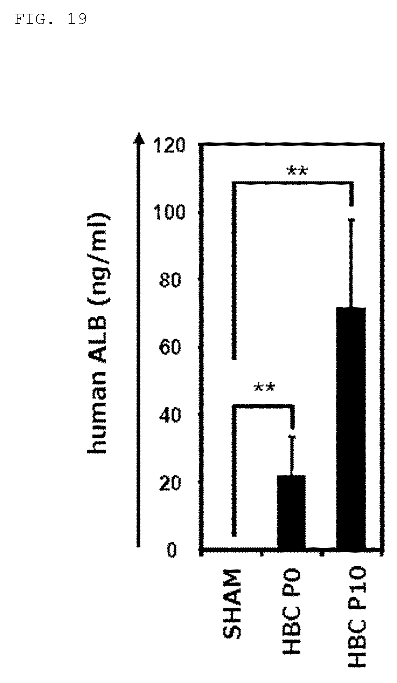

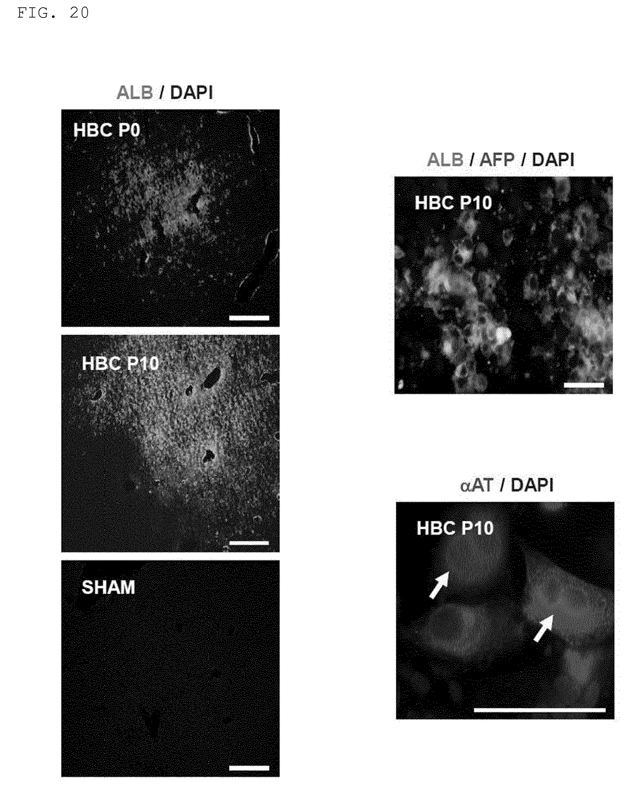

In Example 7, among HBCs derived fromES cells (H9), HBCs without passage (HBC P0) or HBCs passaged 10 times (HBC P10) were suspended in the medium 3 of Reference Example 1. Then, 2×10⁶ cells were transplanted to a CCl₄ (4 mL/kg) -treated Rag2/IL2Rg double-knockout mouse (see FIG. 18). The blood and liver of the mouse were collected two weeks after transplantation. The tissues were fixed with 4% PFA and were used as materials for immunostaining. Further, the secretion of human ALB to the serum was measured by using a commercially available ELISA measuring kit (Bethyl Laboratories, Inc.) (FIG. 19). Further, the expressions of human ALB, human AFP, and human αAT were confirmed by using an immunohistochemical staining method two weeks after transplantation. As a control, nuclear staining (DAPI) was also conducted. As a result, with regard to HBC P10, the expression of human ALB, but not mouse ALB, was confirmed in the liver of the mouse two weeks after transplantation. In addition, the expression of human αAT was also confirmed. Accordingly, it was suggested that HBCs were induced to differentiate into mature hepatocytes also in vivo (FIGS. 20).

### (Example 8) Cell Surface Markers of Cultured Hepatoblast-like Cells

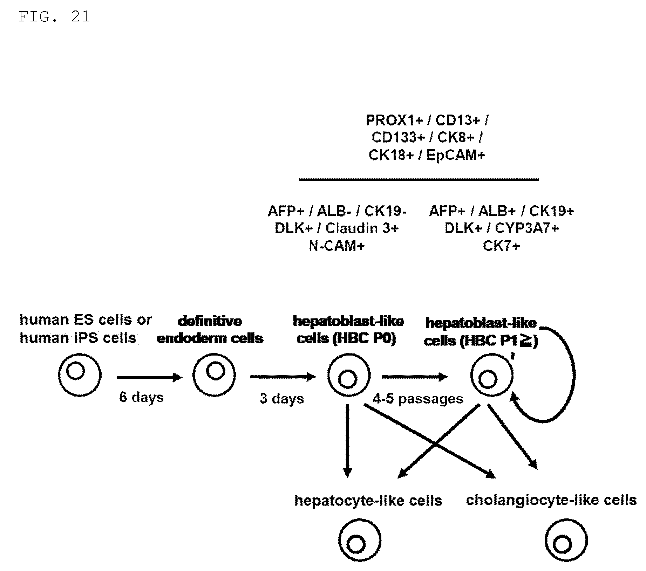

In Example 8, the expressions of various types of markers in HBCs (H9) without passage (HBC P0) and HBCs passaged 1 or more times (HBC P1 and the like) were summarized (FIG. 21). The expressions of various types of markers in HBCs are shown in Table 2.

**[Table 2]**

| | HBC P0 | HBC P1 | HBC P10 | HBC clone |
|---|---|---|---|---|
| AFP | + | + | + | + |
| ALB | - | + | + | + |
| CD13 | + | + | + | + |
| CD133 | + | + | + | + |
| CK19 | - | + | + | + |
| CK7 | - | + | + | + |
| CK8 | + | + | + | + |
| Claudin3 | + | - | - | - |
| CYP3A7 | - | + | + | + |
| DLK1 | + | + | + | + |
| EPCAM | + | + | + | + |
| N-CAM | + | - | - | - |
| PROX1 | + | + | + | + |
| Abilities to undergo induction of differentiation to hepatocytes and cholangiocytes | Bipotent | Bipotent | Bipotent | Bipotent |
| Ability to integrate into liver parenchyma | + | Not examined | + | Not examined |
| Cell diameter (average) | ≥10 µm | ≤10 µm | ≤10 µm | ≤10 µm |
| Nucleus to to cytoplasimc ra | Very high | Intermediate | Intermediate | Intermediate |
| Self-regenerative capacity | None | Having regenerative capacity on LN111 | Having regenerative capacity on LN111 | Having regenerative capacity on LN111 |

### (Example 9) Search for Laminins suitable for maintaining Hepatoblast-like Cells

In Example 9, it was examined whether there was any laminin further suitable for maintaining HBCs with regard to HBCs obtained by the same procedure as in Reference Example 2, by using human ES cells (H9) as a starting material and using the expression of AFP as an indicator.

### The coating treatment was performed on cell culture dishes by using various types of human laminin E8 fragments with different isoforms (laminin 111E8 (LN111E8), laminin 121E8 (LN121E8), laminin 211E8 (LN211E8), laminin 221E8 (LN221E8), laminin 332E8 (LN332E8), laminin 311E8 (LN311E8), laminin 321E8 (LN321E8), laminin 411E8 (LN411E8), laminin 421E8 (LN421E8), laminin 511E8 (LN511E8), and laminin 521E8 (LN521E8)). The coating treatment was conducted by preparing 20 µg/ml of each Laminin Coating Solution, adding the Laminin Coating Solution to each well at 5 µg/cm², leaving cell culture dishes at room temperature for 1 hour or more.

### (1) Expression of AFP in Cultured Hepatoblast-like Cells

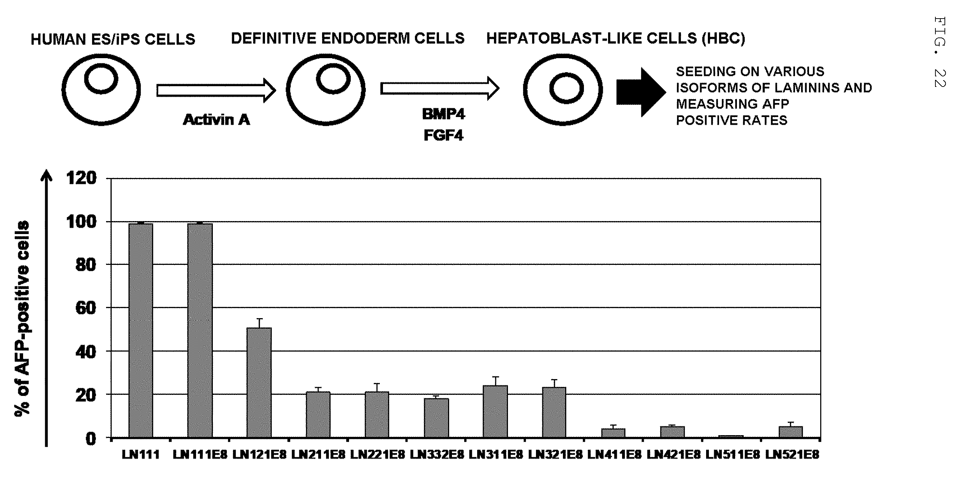

The cells on day 9 obtained by treating cells by the same procedure as in Reference Example 2 were seeded to each well of various types of laminin coated culture dishes at 7.5×10⁴cells/ cm², and after 15 minutes, the medium was exchanged. Cells attached to the dishes were used as HBCs and cells not attached to the dishes were used as NHBCs. The HBCs attached to the dishes were each further cultured for 7 days. Then, the positive rate of AFP (%) of each of the HBCs was confirmed by flow cytometry analysis. As a result, it was confirmed that the cells cultured on a LN111E8-coated culture dish expressed AFP in a similar manner to a group of cells cultured on a LN111-coated culture dish (see FIGS. 22).

### (2) Cell Proliferative Capacity

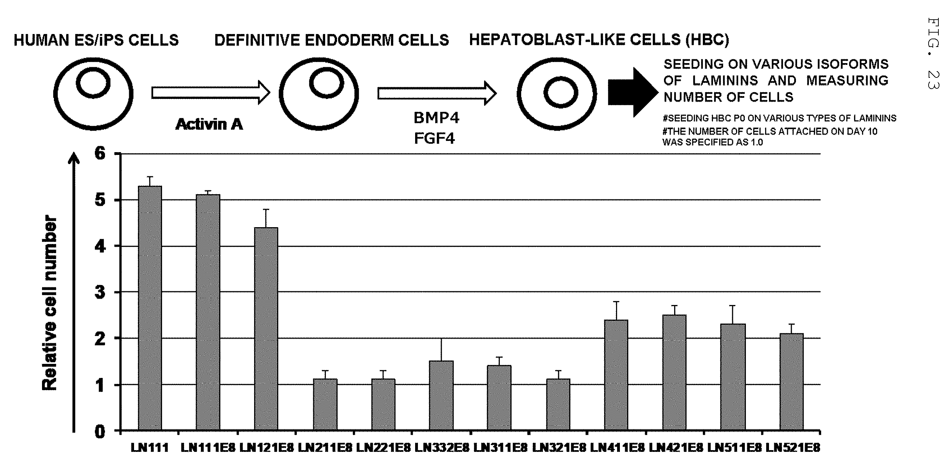

The HBCs attached to the dishes by the above-mentionedmethod were each further cultured for 10 days. At this time, the number of cells was confirmed. As a result, it was confirmed that, when the cells were cultured on a LN111E8-coated culture dish, the cells proliferated to the same degree as the group of cells cultured on a LN111-coated culture dish (see FIGS. 23).

### (Example 10) Properties of Cultured Hepatoblast-like Cells passaged by using LN111- or LN111E8-coated Culture Dish

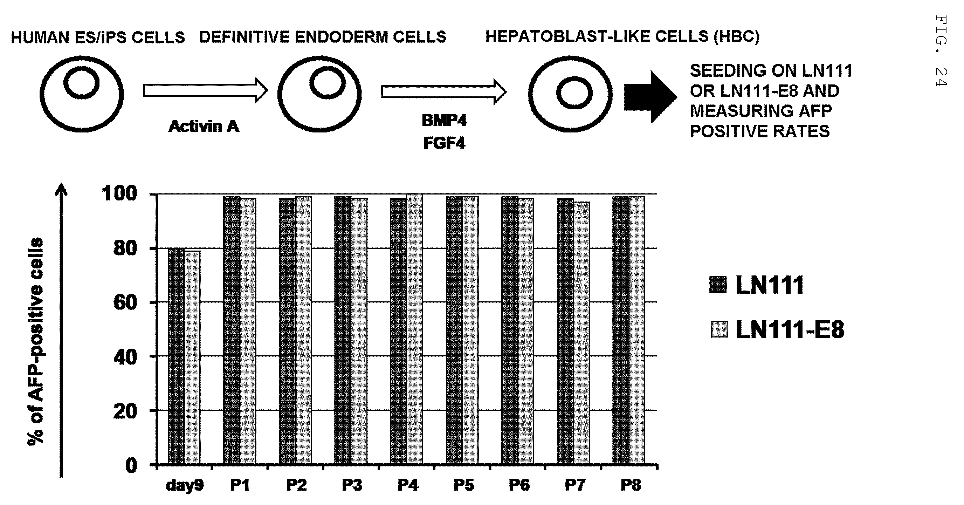

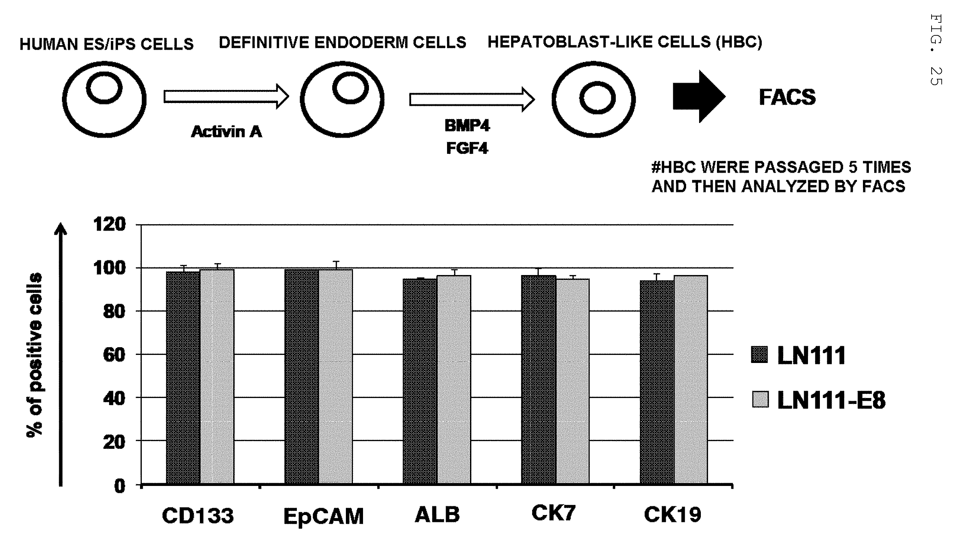

By the same procedure as in Example 9, HBCs were seeded to each well of various types of laminin coated culture dishes at 7.5×10⁴ cells/cm² by using a LN111- or LN111E8-coated culture dish, and the medium was exchanged after 15 minutes (the number of passages of 0, day 9). After that, the cells were cultured for from 6 days to 7 days with the medium being exchanged every day, and a cell suspension was prepared so that the number of cells was 7.5×10⁴ cells/cm². Then, the cells were passaged on the LN111-coated culture dish or the LN111E8-coated culture dish. At the time when such passage operations were repeated 8 times (P8), the positive rate of AFP (%) was confirmed by flow cytometry analysis. As a result, in both LN111- and LN111E8-coating treatments, it was confirmed that the expressions of AFP were maintained (see FIGS. 24). In addition, also with regard to markers other than AFP (CD133, EpCAM, ALB, CK7, and CK19), their positive rates were confirmed by flow cytometry analysis at the time when the passage culture was repeated up to 8 times (P8). As a result, it was confirmed that the expressions of all markers were maintained (see FIGS. 25).

From the above-mentioned results, it was confirmed that the culture product, which was passaged 8 times on LN111- or LN111E8-coated dish, expressed various types of markers and maintained properties of HBCs.

### (Example 11) Expressions of Various Types of Integrins by Hepatoblast-like Cells

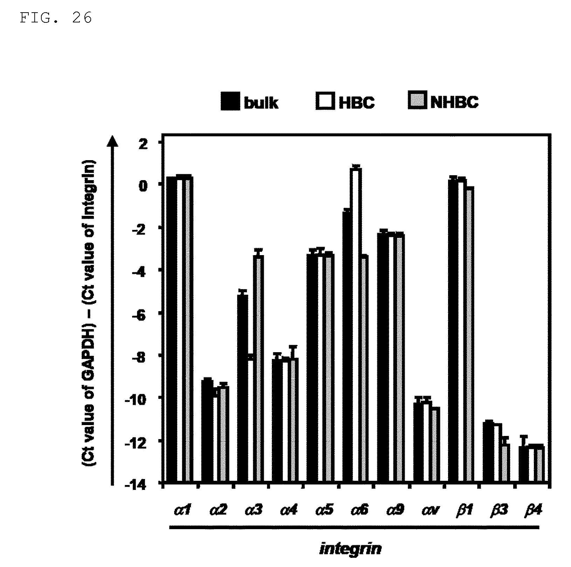

With regard to the HBCs and the NHBCs on day 9 after the differentiation induction treatment which were prepared in Reference Example 2 by using human ES cells (H9) as a starting material, the expressions of various types of integrin genes were confirmed by a real-time RT-PCR method by the same procedure as in Example 1. Various types of commercially available primer sets and reagents were used for real-time RT-PCR measurement, and the measurement was performed by using GAPDH as a housekeeping gene. As a result, it was confirmed that, with regard to the expression of integrin α6, the expression in the HBCs was about 16 times as high as that in the NHBCs, and that, with regard to the expression of integrin α3, the expression in the NHBCs was about 20 times as high as that in the NBCs (FIG. 26).

### Industrial Applicability

### In general, in the case of inducing pluripotent stem cells, such as human ES cells or iPS cells, to differentiate into hepatocytes, a culture period of about 20 days is required. Hitherto, it has been difficult to culture, maintain, and proliferate hepatoblast-like cells derived from iPS cells and/or ES cells.

As described in detail above, the method of the present invention including using a laminin, makes it possible for the first time to culture, maintain, and proliferate hepatoblast-like cells.

The culture product (hepatoblast-like cells) cultured by the method of the present invention can be passaged. In general, passaged differentiation induction intermediate cells are hardly cultured such that these cells proliferate. In the present invention, the number of times of passage culture is not particularly limited, but even when the hepatoblast-like cells are repeatedly passaged at least 1 or more times, for example, 3 or more times, 10 or more times, or 15 or more times, the hepatoblast-like cells can maintain properties of hepatoblast-like cells and can maintain the ability to undergo the induction of differentiation.

The cultured hepatoblast-like cells obtained as described above were positive for AFP, ALB, CK7, andCK19. Further, even when the cultured hepatoblast-like cells were passaged many times, the expressions of integrin α6 and integrin β1 were confirmed. With regard to cultured hepatoblast-like cells which were passaged many times, the expressions of pan-hepatoblast markers CK8, CK18, EpCAM, and CD133, and/or hepatoblast markers AFP, ALB, CYP3A7, and I-CAM, and the like were confirmed. Further, by subjecting the cultured hepatoblast-like cells to further differentiation induction treatment, cells expressing CYP3A4, CYP2C9, CYP2C19, and αAT, which are markers of mature hepatocytes, and cells expressing SOX9 and Type IV collagen, which are markers of bile duct epithelial cells, can be induced. Thus, it was demonstrated that the cultured hepatoblast-like cells was able to be induced to differentiate to mature hepatocytes, bile duct epithelial cells, and the like. In addition, the hepatocytes and the like can be induced to differentiate in vivo by transplanting a substantially pure culture product obtained by the above-mentioned culture method. Accordingly, a culture product obtained by the above-mentioned culture method or mature hepatocytes or bile duct epithelial cells induced from the culture product can be used as a composition for transplantation for use in the regeneration of hepatocytes and/or bile duct epithelial cells.

Desired mature cells, such as mature hepatocytes and bile duct epithelial cells, can be generated in a short time period and the desired number of cells can be acquired at a desired timing by maintaining the hepatoblast-like cells. The hepatocytes, bile duct epithelial cells, and the like thus obtained can be utilized as materials for cell therapy for the regeneration of hepatocytes and/or bile duct epithelial cells. In addition, the in vivo toxicity of a pharmaceutical candidate compound can be predicted beforehand by adding the pharmaceutical candidate compound to hepatocytes, and analyzing the variations in expressions of markers of hepatotoxicity. Accordingly, a pharmaceutical candidate compound which should be excluded due to the problem of toxicity can be screened at an early stage, which leads to an expectation of the acceleration of drug discovery.

## Claims

1. A method of culturing hepatoblast-like cells, the method comprising the following steps of:
bringing a laminin into contact with hepatoblast-like cells generated during a differentiation-inducing process from pluripotent stem cells to hepatocytes; and
removing hepatoblast-like cells not attached to the laminin after bringing the laminin into contact with the hepatoblast-like cells.

2. A method of culturing hepatoblast-like cells according to claim 1, wherein the laminin to be brought into contact with the hepatoblast-like cells generated during the differentiation-inducing process from pluripotent stem cells to hepatocytes is a laminin excluding laminin subtypes of laminin α2β1γ1, lamininaα4β1γ1, and laminin α5β1γ1 among laminin isoforms.

3. Amethod of culturing hepatoblast-like cells according to claim 1, wherein the laminin to be brought into contact with the hepatoblast-like cells generated during the differentiation-inducing process from pluripotent stem cells to hepatocytes comprises a laminin of an isoform having a property of exhibiting an affinity for integrin α6β1 higher than an affinity for integrin α3β1.

4. A method of culturing according to any one of claims 1 to 3, wherein the laminin to be brought into contact with the hepatoblast-like cells generated during the differentiation-inducing process from pluripotent stem cells to hepatocytes comprises a part or whole of laminin α1β1γ1.

5. A method of culturing hepatoblast-like cells according to claim 4, wherein the part of laminin α1β1γ1 comprises at least E8 fragment of laminin α1β1γ1.

6. A method of culturing hepatoblast-like cells according to any one of claims 1 to 5, wherein the hepatoblast-like cells comprise hepatoblast-like cells derived from induced pluripotent stem cells and/or collected embryonic stem cells.

7. A method of culturing hepatoblast-like cells according to claim 6, the method comprising the following steps of:
1) seeding the hepatoblast-like cells derived from induced pluripotent stem cells and/or collected embryonic stem cells on a solid support for cell culture coated with the laminin;
2) removing, in the solid support for cell culture on which the hepatoblast-like cells are seeded, non-adherent hepatoblast-like cells from the solid support; and
3) culturing the hepatoblast-like cells attached to the solid support in the step 2) on the laminin.

8. A method of culturing hepatoblast-like cells according to claim 7, the method further comprising the following steps of:
4) separating the hepatoblast-like cells obtained by culturing in the steps 1) to 3) of claim 7 from the solid support for cell culture, such that the hepatoblast-like cells are suspended in a medium;
5) seeding the suspended cells on a solid support for cell culture coated with the laminin, such that a number of cells is from 2.5×10² cells/cm² to 1.25 × 10⁵ cells/cm², preferably from 2.5 × 10³ cells/cm² to 2.5 × 10⁴ cells/cm², more preferably from 2×10⁴ cells/cm² to 2.5 × 10⁴ cells/cm²; and
6) culturing the hepatoblast-like cells attached to the solid support in the step 5) on the laminin.

9. A method of culturing hepatoblast-like cells according to claim 8, wherein the steps 4) to 6) of claim 8 are repeated at least once.

10. A method of culturing hepatoblast-like cells according to any one of claims 7 to 9, the method comprising:
dispersing the hepatoblast-like cells derived from induced pluripotent stem cells and/or collected embryonic stem cells to single cells, before the step 1) of claim 7 of seeding the hepatoblast-like cells on the solid support for cell culture coated with the laminin; and
seeding the dispersed hepatoblast-like cells on the solid support for cell culture coated with the laminin of the step 1) .

11. A substantially pure culture product of hepatoblast-like cells, comprising cells having an adhesion ability to laminin α1β1γ1 higher than adhesion abilities to laminin α2β1γ1, laminin α4β1γ1, and laminin α5β1γ1 among laminin isoforms.

12. A substantially pure culture product of hepatoblast-like cells, comprising hepatoblast-like cells expressing any one or more kinds of hepatoblast markers selected from ALB, CK19, CK7, and CYP3A7, and expressing integrin α6 and integrin β1.

13. A substantially pure culture product of hepatoblast-like cells, which is a culture product of hepatoblast-like cells expressing an integrin on surfaces thereof, the culture product comprising hepatoblast-like cells expressing integrin α6β1 more dominantly than integrin α3β1.

14. A substantially pure culture product, which is produced by the method of culturing hepatoblast-like cells of any one of claims 1 to 10.

15. A composition for transplantation for use in regeneration of hepatocytes and/or bile duct epithelial cells, the composition comprising the culture product of any one of claims 11 to 14 as an active ingredient.

16. A substrate for culturing hepatoblast-like cells, comprising a solid support for cell culture coated with a part or whole of laminin α1β1γ1.

17. A substrate for culturing hepatoblast-like cells according to claim 16, wherein the part of laminin α1β1γ1 comprises at least E8 fragment of laminin α1β1γ1.

18. A pretreatment method for differentiation induction from hepatoblast-like cells to mature hepatocytes and/or bile duct epithelial cells, the method comprising the following steps of:
bringing a laminin into contact with hepatoblast-like cells generated during a differentiation-inducing process from pluripotent stem cells to hepatocytes; and
removing hepatoblast-like cells not attached to the laminin after bringing the laminin into contact with the hepatoblast-like cells.

19. A pretreatment method for differentiation induction from hepatoblast-like cells to mature hepatocytes and/or bile duct epithelial cells according to claim 18, wherein the laminin to be brought into contact with the hepatoblast-like cells generated during the differentiation-inducing process from pluripotent stem cells to hepatocytes is a laminin excluding laminin subtypes of laminin α2β1γ1, laminin α4β1γ1, and laminin α5β1γ1 among laminin isoforms.

20. A pretreatment method for differentiation induction from hepatoblast-like cells to mature hepatocytes and/or bile duct epithelial cells according to claim 18, wherein the laminin to be brought into contact with the hepatoblast-like cells generated during the differentiation-inducing process from pluripotent stem cells to hepatocytes comprises a laminin of an isoform having a property of exhibiting an affinity for integrin α6β1 higher than an affinity for integrin α3β1.

21. A pretreatment method for differentiation induction from hepatoblast-like cells to mature hepatocytes and/or bile duct epithelial cells according to claim 18, wherein the laminin to be brought into contact with the hepatoblast-like cells generated during the differentiation-inducing process from pluripotent stem cells to hepatocytes comprises a part or whole of laminin α1β1γ1.

22. A pretreatment method for differentiation induction from hepatoblast-like cells to mature hepatocytes and/or bile duct epithelial cells according to claim 21, wherein the part of laminin α1β1γ1 comprises at least E8 fragment of laminin α1β1γ1.
